(19) Europäisches Patentamt
European Patent Office
Office européen des brevets

(11) Veröffentlichungsnummer: **0 106 214**
**B1**

(12)
# EUROPÄISCHE PATENTSCHRIFT

(45) Veröffentlichungstag der Patentschrift: **04.12.85**

(51) Int. Cl.⁴: **C 07 D 417/12**, A 61 K 31/54

(21) Anmeldenummer: **83109512.0**

(22) Anmeldetag: **24.09.83**

(54) **Neue 4-Hydroxy-2H-1,2-benzothiazin-3-carboxamid-1,1-dioxide, Verfahren zu ihrer Herstellung, ihre Verwendung und diese Verbindungen enthaltende Arzneimittel.**

(30) Priorität: **09.10.82 DE 3237473**

(43) Veröffentlichungstag der Anmeldung: **25.04.84 Patentblatt 84/17**

(45) Bekanntmachung des Hinweises auf die Patenterteilung: **04.12.85 Patentblatt 85/49**

(84) Benannte Vertragsstaaten: **AT BE CH DE FR IT LI LU NL SE**

(56) Entgegenhaltungen:
**DE - B - 1 943 265**

**CHEMICAL ABSTRACTS, Band 97, Nr. 15, 11. Oktober 1982, Seite 729, Nr. 127648a, Columbus, Ohio, US**
**PATENTS ABSTRACTS OF JAPAN, Band 6, Nr. 149 (C-118)1027I, 10. August 1982**
**PATENTS ABSTRACTS OF JAPAN, Band 6, Nr. 78 (C-102)956I, 15.05.1982**

(73) Patentinhaber: **Dr. Karl Thomae GmbH, Postfach 1755, D-7950 Biberach (Riss) (DE)**

(72) Erfinder: **Trummlitz, Günter, Dr. Dipl.-Chem., Buchenweg 27, D-7951 Warthausen (DE)**
Erfinder: **Engel, Wolfhard, Dr. Dipl.-Chem., Mozartstrasse 13, D-7950 Biberach 1 (DE)**
Erfinder: **Seeger, Ernst, Dr. Dipl.-Chem., Alpenstrasse 39, D-7950 Biberach 1 (DE)**
Erfinder: **Haarmann, Walter, Dr., Schlierholzweg 27, D-7950 Biberach 1 (DE)**

EP 0 106 214 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue 4-Hydroxy-2H-1,2-benzothiazin-3-carboxamid-1,1-dioxide der allgemeinen Formel I,

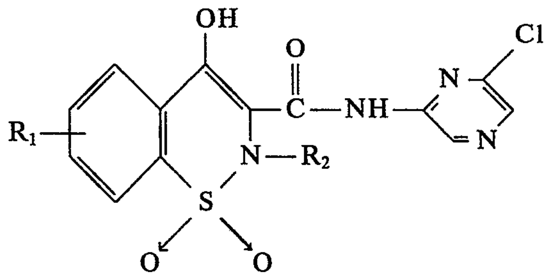

(I)

ihre physiologisch verträglichen Salze mit anorganischen oder organischen Basen, Verfahren zu ihrer Herstellung und sie enthaltende Arzneimittel.

In der obigen allgemeinen Formel I bedeuten:

$R_1$ ein Wasserstoffatom, eine Methyl- oder Methoxygruppe oder ein Fluor- oder Chloratom, und
$R_2$ ein Wasserstoffatom oder eine Methyl-, Äthyl- oder n-Propylgruppe.

Die deutschen Offenlegungsschriften 1 943 265 und 2 756 113 beschreiben 3,4-Dihydro-2H-1,2-benzothiazin-1,1-dioxide bzw. 4-Hydroxy-2H-1,2-benzothiazin-3-carboxamid-1,1-dioxide, die mit den Verbindungen der obigen Formel I verwandt sind. Es hat sich aber überraschenderweise herausgestellt, daß die Verbindungen der allgemeinen Formel I auch gegenüber den konstitutionell am nächsten verwandten Verbindungen dieser Publikation eine andersgeartete pharmakologische Hauptwirkung zeigen. Die dort genannten Verbindungen sind in erster Linie antiphlogistisch wirksam, die Verbindungen der obigen allgemeinen Formel I dagegen stark antithrombotisch.

Die Verbindungen der allgemeinen Formel I lassen sich nach folgenden Verfahren herstellen.

1. Sämtliche Verbindungen der allgemeinen Formel I lassen sich durch Umsetzung von 4-Hydroxy-2H-1,2-benzothiazin-1,1-dioxid-3-carbonsäure-derivaten der allgemeinen Formel II,

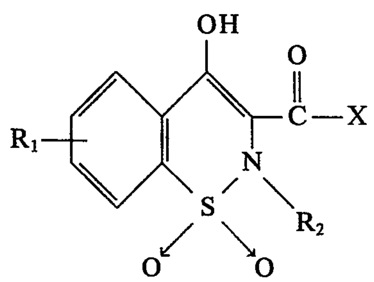

(II)

in der X eine nucleophil austauschbare Gruppe, insbesondere eine Alkoxygruppe mit 1 bis 8 Kohlenstoffatomen, eine Phenylalkoxygruppe mit insgesamt 7 bis 10 Kohlenstoffatomen, die Phenyloxygruppe, ein Halogenatom, die freie Aminogruppe, eine Alkylaminogruppe mit 1 bis 8 Kohlenstoffatomen, eine Cycloalkylaminogruppe mit 3 bis 10 Kohlenstoffatomen, eine Phenylalkylaminogruppe mit insgesamt 7 bis 10 Kohlenstoffatomen oder die Anilinogruppe bedeutet, und $R_1$ und $R_2$ wie oben definiert sind, mit 2-Amino-6-chlor-pyrazin der Formel III

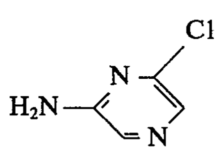

(III)

erhalten.

Die Reaktion der Carbonsäureester der allgemeinen Formel II mit dem 2-Amino-6-chlor-pyrazin der Formel III erfolgt in geeigneten, indifferenten organischen Lösungsmitteln, beispielsweise in aromatischen Kohlenwasserstoffen wie Benzol, Toluol, Xylol, Chlorbenzol, o-Dichlorbenzol oder Tetrahydronaphthalin, in Dimethylformamid, Dimethylacetamid oder Dimethylsulfoxid oder in Hexamethylphosphorsäuretriamid, in Äthern, wie Dimethoxyäthan, Diäthylenglykoldimethyläther oder Diphenyläther oder auch direkt im überschüssigen Pyrazin der Formel III. Man arbeitet bei einer Temperatur von 60 bis 200°C, sofern X in der allgemeinen Formel II eine Alkoxygruppe ist, zwischen 20 und 180°C. Vorzugsweise setzt man in Toluol oder Xylol bei Siedetemperatur um und entfernt, sofern in der allgemeinen Formel II X eine Alkoxygruppe, Phenylalkoxygruppe oder Phenyloxygruppe bedeutet, den bei der Reaktion entstehenden Alkohol oder Phenol durch azeotrope Destillation oder durch Erhitzen unter Rückfluß beispielsweise unter Verwendung eines mit Molekularsieb beschickten Soxhlet-Extraktors. Das Produkt kristallisiert direkt aus dem Reaktionsgemisch aus oder wird bei Verwendung eines mit Wasser mischbaren Lösungsmittels durch Zugabe von Wasser ausgefällt. Ist X in der allgemeinen Formel II die Aminogruppe oder eine, wie oben angegeben, substituierte Aminogruppe, so fügt man bei der Umsetzung vorteilhafterweise noch eine katalytische Menge von p-Toluolsulfonsäure zu und setzt das Pyrazin der Formel III im Überschuß ein. Auch hier kristallisiert das Produkt oft direkt aus dem Reaktionsgemisch aus, man erhält es aber in jedem Fall durch Abdampfen des Lösungsmittels; es kann aber auch bei Verwendung eines mit Wasser mischbaren Lösungsmittels durch Zugabe von Wasser ausgefällt werden.

2. Verbindungen der allgemeinen Formel I, in der $R_2$ eine Methyl-, Äthyl- oder n-Propylgruppe bedeutet und in der $R_1$ die eingangs definierte Bedeutung hat, lassen sich auch durch Umsetzung eines 4-Hydroxy-2H-1,2-benzothiazin-3-carboxamid-1,1-dioxids der allgemeinen Formel IV,

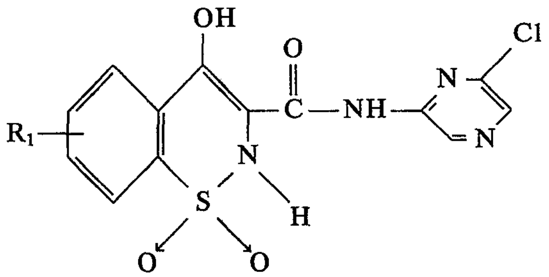

(IV)

in der $R_1$ wie oben definiert ist, mit einem Alkylhalogenid der allgemeinen Formel V,

$$R_{11}—Hal \qquad (V)$$

in der
Hal ein Halogenatom und
$R_{11}$ eine Methyl-, Äthyl- oder n-Propylgruppe bedeutet,
in Gegenwart von Basen, erhalten.

Als Basen können Alkali- oder Erdalkalihydroxide, beispielsweise Natrium-, Kalium- oder Bariumhydroxid oder Alkali- oder Erdalkalicarbonate, wie Natrium- oder Kaliumcarbonat, sowie Alkali- oder Erdalkalimetallalkoholate, beispielsweise Natriummethylat, Kaliumäthylat, Kalium-tert.butylat oder tertiäre Amine, beispielsweise Triäthylamin, eingesetzt werden, sofern man in wäßrigem Medium, in alkoholischem Medium, etwa in Methanol, Äthanol, n-Propanol, iso-Propanol oder in Mischungen aus den genannten Lösungsmitteln arbeitet; im Falle der Anwendung von Alkalialkoholaten arbeitet man am besten in dem entsprechenden alkoholischen Medium.

Das Alkylhalogenid, vorzugsweise ein Alkylbromid oder -jodid, wird zweckmäßigerweise in alkoholischer Lösung direkt zu den übrigen Komponenten in das Reaktionsgemisch gegeben, wobei im Falle des Methylbromids in einer geschlossenen Apparatur gearbeitet wird. Als weitere Lösungsmittel kommen in Frage: Dimethylformamid, Dimethylacetamid, Dimethylsulfoxid, Hexamethylphosphorsäuretriamid.

Sofern man Alkali- oder Erdalkalicarbonate als Basen verwendet, kommen als Lösungsmittel auch aliphatische Ketone, wie Aceton, in Betracht.

Wird die Reaktion in aprotischen organischen Lösungsmitteln, wie z. B. in Benzol oder einem anderen aromatischen Kohlenwasserstoff, in Tetrahydrofuran oder einem anderen offenkettigen oder cyclischen Äther durchgeführt, so kann man als Basen auch Alkalimetallhydride oder Erdalkalimetallhydride, z. B. Natriumhydrid, verwenden. Dabei erfolgt die Zugabe des Alkylhalogenids jedoch erst, wenn sich das Alkalimetallhydrid bzw. Erdalkalimetallhydrid vollständig mit der Ausgangsverbindung der allgemeinen Formel IV umgesetzt hat. Die Reaktionstemperatur beträgt 0 bis 80°C.

In manchen Fällen empfiehlt es sich, vor der Durchführung der beiden vorstehend genannten Verfahren die 4-Hydroxygruppe in Verbindungen der allgemeinen Formeln II oder IV durch eine Schutzgruppe zu schützen, wobei nach der Beendigung der Umsetzung diese Schutzgruppe wieder abgespalten wird. So ist beispielsweise eine Verätherung der 4-Hydroxygruppen vorteilhaft; man führt diese Hydroxygruppen in an sich bekannter Weise in die entsprechenden Alkoxy- oder Phenylalkoxygruppen über, beispielsweise in Alkoxygruppen mit 1 bis 8 Kohlenstoffatomen oder Phenylalkoxygruppen mit insgesamt 7 bis 10 Kohlenstoffatomen und spaltet nach der Umsetzung diese Schutzgruppen, beispielsweise durch Erhitzen in Mineralsäuren, wie Bromwasserstoffsäure, auf Temperaturen bis zu 100°C, oder durch Zugabe von Bortrihalogeniden, wie Bortribromid oder Bortrichlorid, in inerten Lösungsmitteln, wie chlorierten Kohlenwasserstoffen, bei Temperaturen zwischen −80°C und +80°C wieder ab.

Die Verbindungen der allgemeinen Formel I können gewünschtenfalls nach an sich bekannten Methoden in ihre physiologisch verträglichen Salze mit anorganischen oder organischen Basen übergeführt

werden. Als Basen kommen beispielsweise in Betracht:

Alkalialkoholate, Alkalihydroxide, Erdalkalihydroxide, Trialkylammoniumhydroxide, Alkylamine, vorzugsweise Aminopolyalkohole, wie zum Beispiel das N-Methyl-D-glucamin.

Die als Ausgangsverbindungen dienenden Ester der allgemeinen Formel II, in der X einen Alkoxy-, Phenylalkoxy- oder Phenoxyrest bedeutet, sind allgemein bekannt und können zum Beispiel nach der deutschen Offenlegungsschrift 1 943 265 (vgl. auch US-Patentschrift 3 591 584) hergestellt werden; so geht man beispielsweise von den bekannten 3-Oxo-1,2-benzisothiazol-2(3H)-essigsäureester-1,1-dioxiden (Chem. Berichte 30, 1267 [1897] aus und gibt zu diesen ein Alkalimetallalkoholat, beispielsweise Natriumäthanolat, in einem organischen polaren Lösungsmittel wie Dimethylsulfoxid oder Dimethylformamid. Es setzt dabei eine Umlagerungsreaktion ein, wobei nach dem Ansäuern der entsprechende Ester der Formel II erhalten wird, in der $R_2$ Wasserstoff bedeutet. Will man in 2-Stellung dieses Esters die anderen für $R_2$ oben erwähnten Gruppen einführen, so geschieht dies am vorteilhaftesten mittels eines Alkylhalogenids, vorzugsweise mit Hilfe eines Alkyljodids; die Alkylierung erfolgt in Gegenwart einer Base.

Die Ausgangsverbindungen der allgemeinen Formel II, in der X eine Aminogruppe oder substituierte Aminogruppe bedeutet, sind literaturbekannt; sie lassen sich z. B. gemäß den Angaben der deutschen Offenlegungsschrift 1 943 265 (vgl. auch US-Patentschrift Nr. 3 591 584) aus den 4-Hydroxy-2H-1,2-benzothiazin-3-carbon-säureester-1,1-dioxiden der allgemeinen Formel II durch Umsetzung mit Aminen der allgemeinen Formel $NH_2—R_4$, in der $R_4$ ein Wasserstoffatom, eine Alkylgruppe mit 1 bis 8 Kohlenstoffatomen, eine Cycloalkylgruppe mit 3 bis 10 Kohlenstoffatomen, eine Phenylalkylgruppe mit insgesamt 7 bis 10 Kohlenstoffatomen oder die Phenylgruppe bedeutet, in einem indifferenten Lösungsmittel, wie Dimethylsulfoxid oder tert.Butanol, bei Temperaturen zwischen 20 und 200°C herstellen.

Die Ausgangsverbindungen der allgemeinen Formel II, in der X Halogen bedeutet, erhält man beispielsweise durch Umsetzung eines entsprechenden 4-Hydroxy- oder 4-Alkoxy-2H-1,2-benzothiazin-3-carbonsäure-1,1-dioxids mit einem Thionylhalogenid in einem Lösungsmittel, wie Benzol und/oder Dimethylformamid, bei Temperaturen bis zum Rückfluß des Reaktionsgemisches.

Die Verbindung der Formel III ist ebenfalls literaturbekannt.

Die Ausgangsverbindungen der allgemeinen Formel IV stellt man z. B. aus 4-Hydroxy-2H-1,2-benzothiazin-3-carbonsäureester-1,1-dioxiden der allgemeinen Formel II, in der $R_2$ Wasserstoff bedeutet, durch Umsetzung mit 2-Amino-6-chlorpyrazin der Formel III in geeigneten indifferenten organischen Lösungsmitteln bei Temperaturen zwischen 20 und 180°C her.

Wie eingangs erwähnt, besitzen die 4-Hydroxy-2H-1,2-benzothiazin-3-carboxamid-1,1-dioxide der allgemeinen Formel I und ihre physiologisch verträglichen Salze mit anorganischen oder organischen Basen wertvolle pharmakologische Eigenschaften. Diese Verbindungen wirken stark antithrombotisch, ohne die den Substanzen mit antithrombotischer Wirkung allgemein anhaftenden Nebenwirkungen zu zeigen. Die Verbindungen sind daher für den Einsatz als Antithrombotika geeignet.

Es wurde beispielsweise die Substanz

N-(6-Chlor-pyrazin-2-yl)-4-hydroxy-2-methyl-2H-1,2-benzothiazin-3-carboxamid-1,1-dioxid = A

und das

Kaliumsalz des N-(6-Chlor-pyrazin-2-yl)-4-hydroxy-2-methyl-2H-1,2-benzothiazin-3-carboxamid-1,1-dioxids = B

vergleichend mit den bekannten Substanzen

4-Hydroxy-2-methyl-N-(2-pyridyl)-2H-1,2-benzothiazin-3-carboxamid-1,1-dioxid (Piroxicam) = Y

und

4-Hydroxy-2-methyl-N-(2-pyrazinyl)-2H-1,2-benzothiazin-3-carboxamid-1,1-dioxid (vgl. DE-A-1 943 265) = Z

auf ihre Hemmwirkung gegenüber der durch Collagen induzierten Blutplättchen-Aggregation und ihre Blutungszeitverlängerung an der Maus untersucht. Des weiteren wurden die Substanzen auf ihre akute Toxizität geprüft.

a) BORN-Test, Collagen-induzierte Aggregation

Die Thrombozytenaggregation wurde nach der Methode von BORN und CROSS (J. Physiol. 170, 397 [1964]) an plättchenreichem Plasma gesunder Versuchspersonen gemessen. Die Abnahme der

optischen Dichte von Plättchensuspensionen nach Zugabe von Collagen wurde photometrisch gemessen und registriert. Aus dem Neigungswinkel der Dichtekurve wurde auf die Aggregationsgeschwindigkeit geschlossen. Der Punkt der Kurve, bei dem die größte Lichtdurchlässigkeit vorliegt, diente zur Berechnung der »optical density«. Die Collagen-Menge wurde so gewählt, daß sich eine irreversibel verlaufende Kontrollkurve ergibt.

Die angegebenen Zahlen beziehen sich auf die »optical density« und bedeuten prozentuale Änderung der Lichtdurchlässigkeit (= %Abschwächung der Aggregation) unter Substanzeinfluß im Vergleich zur Kontrolle.

Verwendet wurde das handelsübliche Collagen der Firma Hormon-Chemie, München.

Die folgende Tabelle 1 enthält die nach diesem Versuch ermittelten Ergebnisse.

Tabelle 1

| Substanz | Konzentration [Mol/l] | BORN-Test [= % Abschwächung der Aggregation] | $IC_{50}$ [Mol/l] |
|---|---|---|---|
| A | $10^{-5}$ | 95% | $4{,}7 \times 10^{-5}$ |
| | $10^{-6}$ | 71% | |
| | $10^{-7}$ | 6% | |
| Y | $10^{-5}$ | 85% | $2 \times 10^{-6}$ |
| | $10^{-6}$ | 39% | |
| Z | $10^{-5}$ | 15% | $>1 \times 10^{-5}$ |

$IC_{50}$ = 50%ige Abschwächung der Aggregation.

Die Ergebnisse der Tabelle 1 zeigen, daß die Vergleichssubstanzen Y und Z eine 50%ige Abschwächung der Aggregation erst bei einer Konzentration von $2 \times 10^{-6}$ Mol/l bzw. $>1 \times 10^{-5}$ Mol/l erreichen, die Substanz A dagegen die 50%ige Abschwächung bei einer um ca. eine Zehnerpotenz niedrigeren Konzentration bewirkt.

b) BORN-Test, Ratte ex vivo, Collagen-induzierte Aggregation

Untersucht wurden folgende Formen

| | |
|---|---|
| Substanz A | |
| Natriumsalz von A | = C |
| Tetramethylammoniumsalz von A, Substanz B | = D |
| Ethanolammoniumsalz von A | = E |
| Hemicalciumsalz von A | = F |
| Ammoniumsalz von A | = G |

Wachen Ratten im Gewicht von ca. 450 g wurde die Substanz in Form einer wäßrigen Lösung, bei der Substanz A in Form einer Tylosesuspension, per Schlundsonde verabfolgt. Nach 1 Stunde wurde den Tieren (nach vorheriger Nembutal-Narkose) aus der Bauchaorta Blut entnommen. Das per Zentrifugation gewonnene plättchenreiche Plasma wurde gepoolt. Als Kontrolle dienten je 3 Tiere, welche keine Substanz erhalten hatten. Die Art der Blutgewinnung und der Plasmabereitung war in beiden Gruppen die gleiche. Als Anticoagulans wurde 2%iges Natrium-Citrat im Volumenverhältnis 1 + 9 verwendet.

Die Untersuchung erfolgte einheitlich 1 Stunde nach Substanzgabe.

Die Ergebnisse sind in der nachfolgenden Tabelle 2 enthalten.

Tabelle 2

| Dosis mg/kg p.o. | Substanz | | | | | | |
|---|---|---|---|---|---|---|---|
| | A | C | D | B | E | F | G |
| 0,5 | 86% | 100% | – | – | – | – | – |
| 0,25 | 93% | 100% | – | – | – | – | – |
| 0,125 | 14% | 100% | – | – | – | – | – |
| 0,05 | – | 96% | 97% | 100% | 87% | 77% | 92% |
| 0,025 | – | 32% | 20% | 86% | 53% | 64% | 36% |
| 0,0125 | – | 0% | 27% | 26% | 10% | 27% | 7% |

Zahlenangabe: % Aggregationshemmung (Meßparameter: optical density)

Alle untersuchten Salzformen wirkten nach oraler Gabe an der Ratte aggregationshemmend. Alle Salzformen waren hierbei noch erheblich wirksamer als die Substanz A. Bei Verwendung der Salze reichte zur gleichen Aggregationshemmung ca. 1/5 bis 1/6 der Dosis der Substanz A aus.

c) Bestimmung der Blutungszeit bei der Maus

Methode

Die Blutungszeit wird an nicht narkotisierten weiblichen Mäusen von 20–25 g Gewicht nach der Methode von DUKE (J. Amer. Med. Assoc. 15, 1187, 1910) gemessen. Den Tieren wird ca. 0,5 mm von der Schwanzspitze abgeschnitten und das austretende Blut in Abständen von 30 sec vorsichtig mit einem Filterpapierstreifen abgetupft. Die Zahl der so erhaltenen Bluttropfen gibt ein Maß für die Blutungszeit. Die normale Blutungszeit bei der Maus beträgt im Mittel 4,1 min.

Tierstamm: NMRI Biberach
Futter: Altromin R
Anzahl der Tiere pro Versuch: 5

Die Substanzapplikation erfolgt per Schlundsonde 1 Stunde vor der Messung.
Die Ergebnisse sind in der nachfolgenden Tabelle 3 zusammengefaßt:

Tabelle 3

| Substanz | Dosis [mg/kg] | Verlängerung der Blutungszeit |
|---|---|---|
| A | 10 | +103% |
| Y | 10 | +30% |
| Z | 10 | +62% |

Wie man sieht, erreicht die Substanz A eine 100%ige Verlängerung der Blutungszeit bei 10 mg/kg. Bei dieser Dosis zeigen die Vergleichssubstanzen X und Y bereits Nebenwirkungen ohne eine 100%ige Verlängerung der Blutungszeit zu erzielen.

d) Bestimmung der akuten Toxizität

Die akute Toxizität wurde nach oraler Gabe an männlichen und weiblichen Mäusen bestimmt. Die Substanzen wurden als Suspension in Tylose verabreicht.
In der nachfolgenden Tabelle 4 sind die nach den angegebenen Dosen innerhalb von 1, 7 und 14 Tagen gestorbenen Tieren angegeben:

Tabelle 4

| Substanz | Dosis [mg/kg] | Tierzahl | Gestorbene Tiere in der Beobachtungszeit 1 Tag | 7 Tage | 14 Tage |
|---|---|---|---|---|---|
| A | 250 | 10 | 0 | 0 | 0 |
| | 1000 | 10 | 0 | 0 | 0 |
| B | 250 | 5 | 0 | 0 | 0 |
| | 1000 | 5 | 0 | 1 | 1 |
| Y | 250 | 5 | 0 | 3 | 3 |
| Z | 250 | 5 | 1 | 4 | 4 |

Die Substanzen Y und Z wirken stark antiphlogistisch und zwar im Bereich von 1 bis 5 mg/kg p.o. an der Ratte (Ödemtest) und sind stark ulcerogen am Magen der Ratte. Dagegen zeigt die Substanz A keine antiphlogistische Wirkung im Ödemtest ($ED_{35} > 200$ mg/kg) und keine ulcerogene Wirkung bis 100 mg/kg.
Diese nicht vorhandene Nebenwirkung der Substanz A erklärt auch die äußerst geringe akute Toxizität ($\gg 1000$ mg/kg). Die $LD_{50}$-Werte der Vergleichssubstanzen liegen dagegen unter 250 mg/kg.
Die folgenden Beispiele sollen die Erfindung näher erläutern:

## Beispiel 1

N-(6-Chlor-pyrazin-2-yl)-4-hydroxy-2-methyl-2H-1,2-benzothiazin-3-carboxamid-1,1-dioxid

9,0 g (33 mMol) 4-Hydroxy-2-methyl-2H-1,2-benzothiazin-3-carbonsäuremethylester-1,1-dioxid und 4,36 g (33 mMol) 2-Amino-6-chlor-pyrazin wurden in 1200 ml Xylol 24 Stunden am Rückfluß in einer Stickstoffatmosphäre erhitzt. Das dabei entstehende Methanol wurde mit einem 4-Å-Molekularsieb, das sich in einem Soxhlet-Aufsatz befand, entfernt. Nach Abkühlen und Stehen über Nacht wurden die entstandenen Kristalle abfiltriert, die anschließend aus Dioxan umkristallisiert wurden:

7,91 g (64% der Theorie) N-(6-Chlor-pyrazin-2-yl)-4-hydroxy-2-methyl-2H-1,2-benzothiazin-3-carboxamid-1,1-dioxid;
Schmelzpunkt: 278—279°C (Zersetzung);
IR (KBr): 1655 $cm^{-1}$ (CO-Amid)
1H-NMR ([$D_6$]-DMSO): $\delta$ = 11,70 (br.s,1,OH, austauschbar mit $CD_3OD$); 9,3 (s,1,3'-H); 8,6 (s,1,5'-H); 8,1-7,8 (m,4,5-H bis 8-H); 2,85 (s,3,N—$CH_3$).
MS: $M^+$ 366 m/e

$C_{14}H_{11}ClN_4O_4S$ (366,79)

| | C | H | N | Cl | S |
|---|---|---|---|---|---|
| Ber.: | 45,84 | 3,03 | 15,28 | 9,67 | 8,74 |
| Gef.: | 46,08 | 3,04 | 15,31 | 9,62 | 8,64 |

## Beispiel 2

N-(6-Chlor-pyrazin-2-yl)-4-hydroxy-6-methoxy-2-methyl-2H-1,2-benzothiazin-3-carboxamid-1,1-dioxid

5,2 g (17 mMol) 4-Hydroxy-6-methoxy-2-methyl-2H-1,2-benzothiazin-3-carbonsäuremethylester-1,1-dioxid und 2,5 g (19 mMol) 2-Amino-6-chlor-pyrazin wurden in 200 ml Xylol 24 Stunden am Rückfluß erhitzt. Das bei der Reaktion entstehende Methanol wurde mit einem 4-Å-Molekularsieb, das sich in einem Soxhlet-Aufsatz befand, entfernt. Nach Abkühlen auf Raumtemperatur wurde abfiltriert und der Rückstand aus Tetrahydrofuran umkristallisiert:

4,9 g (73% der Theorie) N-(6-Chlor-pyrazin-2-yl)-4-hydroxy-6-methoxy-2-methyl-2H-1,2-benzothiazin-3-carboxamid-1,1-dioxid;
Schmelzpunkt: 289—291°C;
1H-NMR ($CDCl_3$+d—TFA): $\delta$ = 9,45 (s,1,3'-H); 8,55 (s,1,5'-H); 7,93 (d,1,J = 10 Hz, 8-H); 7,62 (d,1,J = 3 Hz, 5-H); 7,35 (dd,1,$J_1$ = 10 Hz, $J_2$ = 3 Hz, 7-H); 4,00 (s,3,$OCH_3$); 3,00 (s,3,$NCH_3$).

$C_{15}H_{13}ClN_4O_5S$ (396,83)

| | | | | | |
|---|---|---|---|---|---|
| Ber.: | C 45,40 | H 3,30 | N 14,12 | Cl 8,93 | S 8,08 |
| Gef.: | 45,70 | 3,58 | 14,00 | 9,08 | 8,33 |

## Beispiel 3

N-(6-Chlor-pyrazin-2-yl)-2,6-dimethyl-4-hydroxy-2H-1,2-benzothiazin-3-carboxamid-1,1-dioxid

2,0 g (7 mMol) 2,6-Dimethyl-4-hydroxy-2H-1,2-benzothiazin-3-carbonsäuremethylester-1,1-dioxid und 1,1 g (8,4 mMol) 2-Amino-6-chlor-pyrazin wurde in 150 ml Xylol analog dem Beispiel 2 umgesetzt und das Reaktionsgemisch aus Äthylenchlorid umkristallisiert:

2,1 g (79% der Theorie) N-(6-Chlor-pyrazin-2-yl)-2,6-dimethyl-4-hydroxy-2H-1,2-benzothiazin-3-carboxamid-1,1-dioxid;
Schmelzpunkt: 296—298°C;
1H-NMR ($CDCl_3$+d—TFA): $\delta$ = 9,55 (s,1,3'-H); 8,55 (s,1,5'-H); 8,05-7,55 (m,3,5-H, 7-H, 8-H); 3,00 (s,3,N—$CH_3$); 2,60 (s,3,$CH_3$).

$C_{15}H_{13}ClN_4O_4S$ (380,83)

| | | | | | |
|---|---|---|---|---|---|
| Ber.: | C 47,31 | H 3,44 | N 14,71 | Cl 9,31 | S 8,42 |
| Gef.: | 47,30 | 3,56 | 14,67 | 9,44 | 8,61 |

## Beispiel 4

N-(6-Chlor-pyrazin-2-yl)-2,7-dimethyl-4-hydroxy-2H-1,2-benzothiazin-3-carboxamid-1,1-dioxid

7,5 g (26 mMol) 2,7-Dimethyl-4-hydroxy-2H-1,2-benzothiazin-3-carbonsäuremethylester-1,1-dioxid und 4,36 g (33 mMol) 2-Amino-6-chlor-pyrazin wurden in 400 ml Xylol analog dem Beispiel 2 umgesetzt und aufgearbeitet:

7,2 g (73% der Theorie) N-(6-Chlor-pyrazin-2-yl)-2,7-dimethyl-4-hydroxy-2H-1,2-benzothiazin-3-carboxamid-1,1-dioxid;
IR (KBr): 1645 $cm^{-1}$ (CO-Amid)

$C_{15}H_{13}ClN_4O_4S$ (380,83)

| | | | | | |
|---|---|---|---|---|---|
| Ber.: | C 47,31 | H 3,44 | N 14,71 | Cl 9,31 | S 8,42 |
| Gef.: | 47,09 | 3,52 | 14,70 | 9,45 | 8,30 |

## Beispiel 5

N-(6-Chlor-pyrazin-2-yl)-7-fluor-4-hydroxy-2-methyl-2H-1,2-benzothiazin-3-carboxamid-1,1-dioxid

4,1 g (15 mMol) 7-Fluor-4-hydroxy-2-methyl-2H-1,2-benzothiazin-3-carbonsäuremethylester-1,1-dioxid und 2,3 g (18 mMol) 2-Amino-6-chlorpyrazin wurden in 200 ml Xylol analog dem Beispiel 2 umgesetzt und analog diesem Beispiel aufgearbeitet:

3,8 g (66% der Theorie) N-(6-Chlor-pyrazin-2-yl)-7-fluor-4-hydroxy-2-methyl-2H-1,2-benzothiazin-3-carboxamid-1,1-dioxid;
IR (KBr): 1645 $cm^{-1}$ (CO-Amid)

$C_{14}H_{10}ClFN_4O_4S$ (384,78)

| | | | | |
|---|---|---|---|---|
| Ber.: | C 43,70 | H 2,62 | N 14,56 | S 8,33 |
| Gef.: | 43,84 | 2,89 | 14,39 | 8,30 |

## Beispiel 6

### 6-Chlor-N-(6-chlor-pyrazin-2-yl)-4-hydroxy-2-methyl-2H-1,2-benzothiazin 3-carboxamid-1,1-dioxid

3,04 g (10 mMol) 6-Chlor-4-hydroxy-2-methyl-2H-1,2-benzothiazin-3-carbonsäuremethylester-1,1-dioxid und 1,5 g (12 mMol) 2-Amino-6-chlor-pyrazin wurden in 150 ml Xylol analog dem Beispiel 2 umgesetzt. Es wurden 3,2 g (80% der Theorie) 6-Chlor-N-(6-chlor-pyrazin-2-yl)-4-hydroxy-2-methyl-2H-1,2-benzothiazin-3-carboxamid-1,1-dioxid erhalten;
Schmelzpunkt: 283—284°C (aus Dioxan).

$C_{14}H_{10}Cl_2N_4O_4S$ (401,23)

| | | | | | |
|---|---|---|---|---|---|
| Ber.: | C 41,91 | H 2,51 | N 13,96 | Cl 17,67 | S 7,99 |
| Gef.: | 42,05 | 2,62 | 14,09 | 17,54 | 7,80 |

## Beispiel 7

### N-(6-Chlor-pyrazin-2-yl)-4-hydroxy-2H-1,2-benzothiazin-3-carboxamid-1,1-dioxid

5,1 g (20 mMol) 4-Hydroxy-2H-1,2-benzothiazin-3-carbonsäuremethylester-1,1-dioxid und 3,0 g (23 mMol) 2-Amino-6-chlorpyrazin wurden in 400 ml Xylol 12 Stunden am Rückfluß unter Stickstoffatmosphäre erhitzt. Das dabei entstehende Methanol wurde mit einem 4-Å-Molekularsieb, das sich in einem Soxhlet-Aufsatz befand, entfernt. Nach dem Abkühlen wurde das Reaktionsgemisch eingeengt und der Rückstand wurde über eine Kieselgelsäure chromatographisch gereinigt und ergab 2,3 g (33% der Theorie) N-(6-Chlor-pyrazin-2-yl)-4-hydroxy-2H-1,2-benzothiazin-3-carboxamid-1,1-dioxid;
Schmelzpunkt: 234—235°C (aus Ethanol).

$C_{13}H_9ClN_4O_4S$ (352,76)

| | | | | | |
|---|---|---|---|---|---|
| Ber.: | C 44,26 | H 2,57 | N 15,88 | Cl 10,05 | S 9,09 |
| Gef.: | 44,02 | 2,65 | 15,92 | 10,10 | 9,24 |

## Beispiel 8

### N-(6-Chlor-pyrazin-2-yl)-2-ethyl-4-hydroxy-2H-1,2-benzothiazin-3-carboxamid-1,1-dioxid

8,5 g (30 mMol) 2-Ethyl-4-hydroxy-2H-1,2-benzothiazin-3-carbonsäuremethylester-1,1-dioxid und 3,9 g (30 mMol) 2-Amino-6-chlor-pyrazin wurden in 600 ml Xylol analog dem Beispiel 1 umgesetzt und aufgearbeitet und ergaben 7,3 g (64% der Theorie) N-(6-chlor-pyrazin-2-yl)-2-ethyl-4-hydroxy-2H-1,2-benzothiazin-3-carboxamid-1,1-dioxid;
Schmelzpunkt: 233—235°C (aus Xylol).

$C_{15}H_{13}ClN_4O_4S$ (380,83)

| | | | | | |
|---|---|---|---|---|---|
| Ber.: | C 47,31 | H 3,44 | Cl 9,31 | N 14,71 | S 8,42 |
| Gef.: | 47,42 | 3,44 | 9,50 | 14,62 | 8,51 |

## Beispiel 9

### N-(6-Chlor-pyrazin-2-yl)-4-hydroxy-2-n-propyl-2H-1,2-benzothiazin-3-carboxamid-1,1-dioxid

2,97 g (10 mMol) 2-n-Propyl-4-hydroxy-2H-1,2-benzothiazin-3-carbonsäuremethylester-1,1-dioxid und 1,3 g (10 mMol) 2-Amino-6-chlor-pyrazin wurden in 150 ml Xylol analog dem Beispiel 1 umgesetzt und ergaben 2,05 g (52% der Theorie) N-(6-Chlor-pyrazin-2-yl)-4-hydroxy-2-n-propyl-2H-1,2-benzothiazin-3-carboxamid-1,1-dioxid.

$C_{16}H_{15}ClN_4O_4S$ (394,86)

| | | | | | |
|---|---|---|---|---|---|
| Ber.: | C 48,67 | H 3,83 | Cl 8,98 | N 14,19 | S 8,12 |
| Gef.: | 48,91 | 3,79 | 8,90 | 14,18 | 8,03 |

Beispiel 10

a) Natriumsalz des N-(6-Chlor-pyrazin-2-yl)-4-hydroxy-2-methyl-2H-1,2-benzothiazin-3-carboxamid-1,1-dioxids

1 g (2,7 mMol) N-(6-Chlor-pyrazin-2-yl)-4-hydroxy-2-methyl-2H-1,2-benzothiazin-3-carboxamid-1,1-dioxid wurde in 2,7 ml einer 1N-Natronlauge und 50 ml Ethanol gelöst. Die Lösung wurde eingeengt und der Rückstand wurde aus Essigester/Ethylenchlorid (1 : 1) umkristallisiert: 900 mg (86% der Theorie) Natriumsalz;
Schmelzpunkt: 214—215°C.

$C_{14}H_{10}ClN_4NaO_4S$ (388,78)

| | | | | | |
|---|---|---|---|---|---|
| Ber.: | C 43,25 | H 2,59 | Cl 9,12 | N 14,41 | S 8,25 |
| Gef.: | 42,90 | 2,84 | 9,14 | 14,09 | 8,10 |

b) Kaliumsalz des N-(6-Chlor-pyrazin-2-yl)-4-hydroxy-2-methyl-2H-1,2-benzothiazin-3-carboxamid-1,1-dioxids

36,4 g N-(6-Chlor-pyrazin-2-yl)-4-hydroxy-2-methyl-2H-1,2-benzothiazin-3-carboxamid-1,1-dioxid werden in 0,5 l Ethanol suspendiert und mit 99,2 ml 1N-Kalilauge versetzt. Die Lösung wurde vollständig zur Trockne eingeengt, der Rückstand in einem Gemisch von 800 ml Tetrahydrofuran und 50 ml Wasser am Rückfluß in Lösung gebracht und heiß filtriert. Nach Abkühlen auf Labortemperatur wurde die Lösung im Eisschrank bei 0°C über Nacht gekühlt und vom Niederschlag abfiltriert. Der Niederschlag wurde auf der Nutsche mit reichlich Ether gewaschen und zunächst 1 Stunde im Exsiccator über Paraffin, danach 2 Stunden im Trockenschrank bei 60°C getrocknet.
Schmelzpunkt: 234—236°C (Zersetzung).

$C_{14}H_{10}ClN_4KO_4S \cdot H_2O$ (422,90)

| | | | | | |
|---|---|---|---|---|---|
| Ber.: | C 39,76 | H 2,86 | Cl 8,76 | N 13,84 | S 7,92 |
| Gef.: | 39,84 | 2,80 | 8,39 | 13,43 | 7,68 |

Beispiel 11

N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-2H-1,2-benzothiazin-3-carbonsäureäthylester-1,1-dioxid und 2-Amino-6-chlor-pyrazin analog dem Beispiel 1; Ausbeute 60% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 45,91 | 3,09 | 9,60 | 15,00 | 8,78 |

Beispiel 12

N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-2H-1,2-benzothiazin-3-carbonsäuremethylester-1,1-dioxid und 2-Amino-6-chlor-pyrazin analog dem Beispiel 1, aber unter Verwendung von o-Dichlorbenzol als Lösungsmittel; Ausbeute 48% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 45,91 | 3,09 | 9,60 | 15,00 | 8,78 |

## Beispiel 13

N-(6-Chlor-pyrazin-2-yl)-4-hydroxy-2-methyl-2H-1,2-benzothiazin-3-carboxamid-1,1-dioxid

1,23 g (4,5 mMol) 4-Hydroxy-2-methyl-2H-1,2-benzothiazin-3-carbonsäurechlorid-1,1-dioxid wurden in 10 ml Dimethylformamid gelöst und portionsweise mit 1,3 g (10 mMol) 2-Amino-6-chlor-pyrazin versetzt. Das Reaktionsgemisch wurde 24 Stunden bei Raumtemperatur gerührt und anschließend mit 40 ml Wasser versetzt. Es wurde 20 Minuten bei Raumtemperatur gerührt und danach wurde der Niederschlag abfiltriert, gewaschen und getrocknet. Umkristallisation aus Dioxan ergab 0,4 g (24% der Theorie) N-(6-Chlor-pyrazin-2-yl)-4-hydroxy-2-methyl-2H-1,2-benzothiazin-3-carboxamid-1,1-dioxid;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 46,02 | 3,00 | 9,72 | 15,42 | 8,84 |

## Beispiel 14

N-(6-Chlor-pyrazin-2-yl)-4-hydroxy-2-methyl-2H-1,2-benzothiazin-3-carboxamid-1,1-dioxid

1,0 g (3 mMol) 4-Hydroxy-2-methyl-N-phenyl-2H-1,2-benzothiazin-3-carboxamid-1,1-dioxid wurden mit 1,3 g (10 mMol) 2-Amino-6-chlor-pyrazin und 0,1 g p-Toluolsulfonsäure in 250 ml Xylol 72 Stunden am Rückfluß erhitzt. Nach dem Abkühlen wurde das Reaktionsgemisch mit 2 N Salzsäure und anschließend mit Wasser gewaschen, getrocknet und im Vakuum eingeengt. Der verbleibende Rückstand wurde säulenchromatographisch (Merck Kieselgel 60; Korngröße: 0,2—0,5 mm; Elutionsmittel: Chloroform/Äthanol; 90 : 10) gereinigt und ergab 0,25 g (23% der Theorie) N-(6-Chlor-pyrazin-2-yl)-4-hydroxy-2-methyl-2H-1,2-benzothiazin-3-carboxamid-1,1-dioxid;
Schmelzpunkt: 278—279°C (Zersetzung) aus Dioxan.

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 45,50 | 3,09 | 9,70 | 15,20 | 8,79 |

## Beispiel 15

a) N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-2H-1,2-benzothiazin-3-carboxamid-1,1-dioxid und 2-Amino-6-chlor-pyrazin und p-Toluolsulfonsäure analog dem Beispiel 14 in einer Ausbeute von 64% der Theorie;
Schmelzpunkt: 278—279°C (aus Dioxan).

b) N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-N-methyl-2H-1,2-benzothiazin-3-carboxamid-1,1-dioxid und 2-Amino-6-chlor-pyrazin und p-Toluolsulfonsäure analog Beispiel 14 in einer Ausbeute von 68% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 45,61 | 3,14 | 9,71 | 15,02 | 8,58 |

c) N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-N-ethyl-2H-1,2-benzothiazin-3-carboxamid-1,1-dioxid und 2-Amino-6-chlor-pyrazin und p-Toluolsulfonsäure analog Beispiel 14 in einer Ausbeute von 68% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 45,62 | 3,11 | 9,70 | 15,04 | 8,59 |

d) N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-N-cyclohexyl-2H-1,2-benzothiazin-3-carboxamid-1,1-dioxid und 2-Amino-6-chlor-pyrazin und p-Toluolsulfonsäure analog Beispiel 14 in einer Ausbeute von 68% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 45,68 | 3,04 | 9,69 | 15,12 | 8,60 |

Beispiel 16

2-Äthyl-N-(6-chlor-pyrazin-2-yl)-4-hydroxy-2H-1,2-benzothiazin-3-carboxamid-1,1-dioxid

Zu einer Lösung von 0,7 g (2 mMol) N-(6-Chlor-pyrazin-2-yl)-4-hydroxy-2H-1,2-benzothiazin-3-carboxamid-1,1-dioxid in 30 ml Methanol und 2,0 ml 1 N Natronlauge wurden 0,94 g (6 mMol) Äthyljodid gegeben. Das Reaktionsgemisch wurde 24 Stunden bei Raumtemperatur gerührt und danach neutralisiert und im Vakuum eingeengt. Der Rückstand wurde säulenchromatographisch (Merck Kieselgel 60, Korngröße 0,2—0,5 mm; Elutionsmittel: Chloroform/Äthanol 90 : 10) gereinigt und ergab nach Umkristallisation aus Xylol 0,35 g (46% der Theorie) 2-Äthyl-4-hydroxy-N-(6-chlor-pyrazin-2-yl)-2H-1,2-benzothiazin-3-carboxamid-1,1-dioxid;
Schmelzpunkt: 233—235°C (Zersetzung) aus Xylol.
Der Ersatz der Natronlauge durch Kalilauge, Natriummethylat oder Kalium-tert.butylat erbrachte ähnliche Ausbeuten (zwischen 40 und 50%).

$C_{15}H_{13}ClN_4O_4S$ (380,83)

| | | | | | |
|---|---|---|---|---|---|
| Ber.: | C 47,31 | H 3,44 | N 14,71 | S 8,42 | Cl 9,31 |
| Gef.: | 47,07 | 3,48 | 14,50 | 8,40 | 9,07 |

Beispiel 17

N-(6-Chlor-pyrazin-2-yl)-4-hydroxy-2-methyl-2H-1,2-benzothiazin-3-carboxamid-1,1-dioxid

Hergestellt aus N-(6-Chlor-pyrazin-2-yl)-4-hydroxy-2H-1,2-benzothiazin-3-carboxamid-1,1-dioxid und Methyljodid analog Beispiel 16 in einer Ausbeute von 64% der Theorie; bei Verwendung von Äthanol als Lösungsmittel von 43% der Theorie.
Schmelzpunkt: 278—279°C (Zersetzung) aus Dioxan.

$C_{14}H_{11}ClN_4O_4S$ (366,79)

| | | | | | |
|---|---|---|---|---|---|
| Ber.: | C 45,84 | H 3,03 | N 9,67 | S 8,74 | Cl 9,67 |
| Gef.: | 45,60 | 3,00 | 9,72 | 8,70 | 9,90 |

Beispiel 18

Ethanolaminsalz des N-(6-Chlorpyrazin-2-yl)-4-hydroxy-2-methyl-2H-1,2-benzothiazin-3-carboxamid-1,1-dioxids

Eine Suspension von N-(6-Chlor-pyrazin-2-yl)-4-hydroxy-2-methyl-2H-1,2-benzothiazin-3-carboxamid-1,1-dioxid in Wasser wurde mit einem Moläquivalent Ethanolamin versetzt. Die Lösung wurde eingeengt und aus Wasser umkristallisiert;
Schmelzpunkt: 225—226°C (Zersetzung).

$C_{16}H_{18}ClN_5O_5S$ (427,89)

| | | | | | |
|---|---|---|---|---|---|
| Ber.: | C 44,91 | H 4,24 | Cl 8,29 | N 16,37 | S 7,49 |
| Gef.: | 45,18 | 4,28 | 8,23 | 16,65 | 7,42 |

Analog dem Beispiel 18 wurden hergestellt:

Calciumsalz des N-(6-Chlor-pyrazin-2-yl)-4-hydroxy-2-methyl-2H-1,2-benzothiazin-3-carboxamid-1,1-dioxids;
Schmelzpunkt: 246—248°C
Tetramethylammoniumsalz des N-(6-Chlor-pyrazin-2-yl)-4-hydroxy-2-methyl-2H-1,2-benzothiazin-3-carboxamid-1,1-dioxids;
Schmelzpunkt: 216°C (Zersetzung)
und
Ammoniumsalz des N-(6-Chlor-pyrazin-2-yl)-4-hydroxy-2-methyl-2H-1,2-benzothiazin-3-carboxamid-1,1-dioxids;
Schmelzpunkt: 273°C (Zersetzung).

## Beispiel 19

a) N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-2H-1,2-benzothiazin-3-carbonsäurepropylester-1,1-dioxid und 2-Amino-6-chlor-pyrazin analog dem Beispiel 1; Ausbeute 73% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 45,90 | 3,29 | 9,68 | 15,09 | 8,71 |

b) N-(6-Chlor-pyrazin-2-yl)-4-hydroxy-2-methyl-2H-1,2-benzothiazin-3-carboxamin-1,1-dioxid

Hergestellt aus 4-Hydroxy-2-methyl-2H-1,2-benzothiazin-3-carbonsäurebutylester-1,1-dioxid und 2-Amino-6-chlor-pyrazin analog dem Beispiel 1; Ausbeute 58% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 46,01 | 3,01 | 9,60 | 15,51 | 8,70 |

c) N-(6-Chlor-pyrazin-2-yl)-4-hydroxy-2-methyl-2H-1,2-benzothiazin-3-carboxamid-1,1-dioxid

Hergestellt aus 4-Hydroxy-2-methyl-2H-1,2-benzothiazin-3-carbonsäurebenzylester-1,1-dioxid und 2-Amino-6-chlor-pyrazin analog dem Beispiel 1; Ausbeute 49% der Theorie;
Schmelzpunkt: 278—279°C (Zersetzung).

$C_{14}H_{11}ClN_4O_4S$ (366,79)

| | | | | | |
|---|---|---|---|---|---|
| Ber.: | C 45,84 | H 3,03 | Cl 9,67 | N 15,28 | S 8,74 |
| Gef.: | 46,10 | 3,16 | 9,78 | 15,01 | 8,56 |

Die neuen Verbindungen der allgemeinen Formel I lassen sich zur pharmazeutischen Anwendung in die üblichen pharmazeutischen Zubereitungsformen einarbeiten. Die Einzeldosis beträgt bei Erwachsenen 10 bis 100 mg, die Tagesdosis 20 bis 200 mg.

Die nachfolgenden Beispiele beschreiben die Herstellung einiger pharmazeutischer Zubereitungsformen:

## Beispiel I

Tabletten mit 25 mg N-(6-Chlor-pyrazin-2-yl)-4-hydroxy-2-methyl-2H-1,2-benzothiazin-3-carboxamid-1,1-dioxid

Zusammensetzung:

1 Tablette enthält:

| | |
|---|---|
| Wirksubstanz | 25,0 mg |
| Maisstärke | 97,0 mg |

| | |
|---|---|
| Polyvinylpyrrolidon | 175,0 mg |
| Magnesiumstearat | 3,0 mg |
| | 300,0 mg |

Herstellungsverfahren

Die Mischung der Wirksubstanz mit Maisstärke wird mit einer 14%igen Lösung des Polyvinylpyrrolidons in Wasser durch ein Sieb mit 1,5 mm Maschenweite granuliert, bei 45°C getrocknet und nochmals durch obiges Sieb gerieben. Das so erhaltene Granulat wird mit Magnesiumstearat gemischt und zu Tabletten verpreßt.

| | |
|---|---|
| Tablettengewicht: | 300 mg |
| Stempel: | 10 mm, flach. |

Beispiel II

Dragées mit 25 mg N-(6-Chlor-pyrazin-2-yl)-4-hydroxy-2-methyl-2H-1,2-benzothiazin-3-carboxamid-1,1-dioxid

Zusammensetzung:

1 Dragéekern enthält:

| | |
|---|---|
| Wirksubstanz | 25,0 mg |
| Maisstärke | 205,0 mg |
| Gelatine | 8,0 mg |
| Talk | 18,0 mg |
| Magnesiumstearat | 44,0 mg |
| | 300,0 mg |

Herstellungsverfahren:

Die Mischung der Wirksubstanz mit Maisstärke wird mit einer 10%igen wäßrigen Gelatine-Mischung durch ein Sieb der Maschenweite 1,5 mm granuliert, bei 45°C getrocknet und nochmals durch obiges Sieb gerieben. Das so erhaltene Granulat wird mit Talk und Magnesiumstearat gemischt und zu Dragéekernen verpreßt.

| | |
|---|---|
| Kerngewicht: | 300,0 mg |
| Stempel: | 10 mm, gewölbt |

Die Dragéekerne werden nach bekannten Verfahren mit einer Hülle überzogen, die im wesentlichen aus Zucker und Talkum besteht. Die fertigen Dragées werden mit Hilfe von Bienenwachs poliert.

| | |
|---|---|
| Dragéegewicht: | 580 mg. |

Beispiel III

Gelatine-Kapseln mit 25 mg N-(6-Chlor-pyrazin-2-yl)-4-hydroxy-2-methyl-2H-1,2-benzothiazin-3-carboxamid-1,1-dioxid

Zusammensetzung:

1 Gelatine-Kapsel enthält:

| | |
|---|---|
| Wirksubstanz | 25,0 mg |
| Maisstärke | 365,0 mg |
| Aerosil | 6,0 mg |
| Magnesiumstearat | 4,0 mg |
| | 400,0 mg |

Herstellungsverfahren

Die Substanzen werden intensiv gemischt und in Gelatine-Kapseln Größe 1 abgefüllt.

| | |
|---|---|
| Kapselinhalt: | 400 mg |

Beispiel IV

Suppositorien mit 25 mg N-(6-Chlor-pyrazin-2-yl)-4-hydroxy-2-methyl-2H-1,2-benzothiazin-3-carboxamid-1,1-dioxid

Zusammensetzung:

1 Zäpfchen enthält:

| | |
|---|---|
| Wirksubstanz | 25,0 mg |
| Suppositorienmassen (z. B. Witepsol W 45) | 1725,0 mg |
| | 1750,0 mg |

Herstellungsverfahren

Die feinpulverisierte Wirksubstanz wird mit Hilfe eines Eintauchhomogenisators in die geschmolzene und auf 40°C abgekühlte Zäpfchenmasse eingerührt. Die Masse wird bei 38°C in leicht vorgekühlte Formen gegossen.

| | |
|---|---|
| Zäpfchengewicht: | 1,75 g |

Beispiel V

Suspension mit 25 mg N-(6-Chlor-2-pyrazinyl)-4-hydroxy-2-methyl-2H-1,2-benzothiazin-3-carboxamid-1,1-dioxid in 5 ml

Zusammensetzung:

| | |
|---|---|
| Wirksubstanz | 0,5 g |
| Dioctylnatriumsulfosuccinat (DONSS) | 0,02 g |
| Benzoesäure | 0,1 g |
| Natriumcyclamat | 0,2 g |
| Aerosil® | 1,0 g |
| Polyvinylpyrrolidon | 0,1 g |
| Glycerin | 25,0 g |
| Grapefruit-Aroma | 0,1 g |
| Dest. Wasser ad | 100,0 ml |

Herstellungsverfahren

In dem auf 70°C erwärmten Wasser werden nacheinander DONSS, Benzoesäure, Natriumcyclamat und Polyvinylpyrrolidon gelöst. Man gibt Glycerin und Aerosil dazu, kühlt auf Raumtemperatur ab und suspendiert mit Hilfe eines Eintauchhomogenisators die feinpulverisierte Wirksubstanz. Anschließend wird aromatisiert und mit Wasser auf das angegebene Volumen aufgefüllt. 5 ml Suspension enthalten 25 mg Wirksubstanz.

**Patentansprüche für die Vertragsstaaten: BE, CH, DE, FR, IT, LI, LU, NL, SE**

1. 4-Hydroxy-2H-1,2-benzothiazin-3-carboxamid-1,1-dioxide der allgemeinen Formel I,

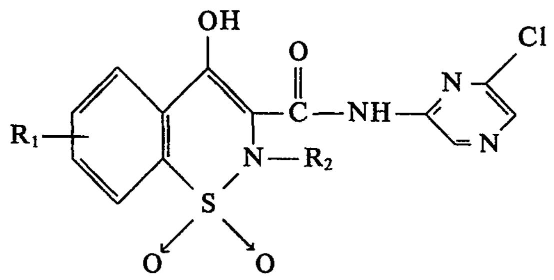

(I)

in der

$R_1$ ein Wasserstoffatom, die Methyl- oder Methoxygruppe oder ein Fluor- oder Chloratom und
$R_2$ ein Wasserstoffatom oder eine Methyl-, Äthyl- oder n-Propylgruppe bedeuten,

und deren physiologisch verträglichen Salze mit anorganischen oder organischen Basen.

2. N-(6-Chlor-pyrazin-2-yl)-4-hydroxy-2-methyl-2H-1,2-benzothiazin-3-carboxamid-1,1-dioxid und dessen physiologisch verträglichen Salze mit anorganischen oder organischen Basen.

3. Die Natriumsalze der 4-Hydroxy-2H-1,2-benzothiazin-3-carboxamid-1,1-dioxide der allgemeinen Formel I gemäß Anspruch 1.

4. Verfahren zur Herstellung von 4-Hydroxy-2H-1,2-benzothiazin-3-carboxamid-1,1-dioxiden gemäß Anspruch 1 und von deren Salzen mit anorganischen oder organischen Basen, dadurch gekennzeichnet, daß

a) ein 4-Hydroxy-2H-1,2-benzothiazin-1,1-dioxid-3-carbonsäurederivat der allgemeinen Formel II,

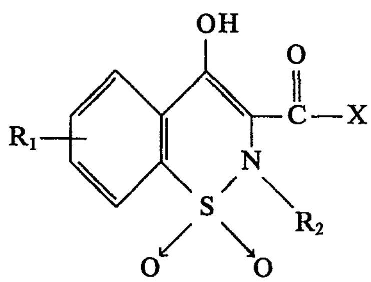

(II)

in der X eine nucleophil austauschbare Gruppe ist und $R_1$ und $R_2$ wie oben definiert sind, mit 2-Amino-6-chlor-pyrazin der Formel III

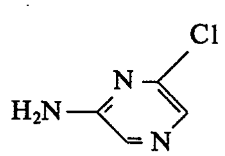

(III)

in einem indifferenten organischen Lösungsmittel oder in einem Überschuß des Pyrazins der Formel III bei Temperaturen zwischen 20 und 200°C umgesetzt wird oder

b) zur Herstellung von Verbindungen der allgemeinen Formel I, in der $R_2$ die Methyl-, Äthyl- oder n-Propylgruppe bedeutet und $R_1$ wie eingangs definiert ist, ein 4-Hydroxy-2H-1,2-benzothiazin-3-carboxamid der allgemeinen Formel IV,

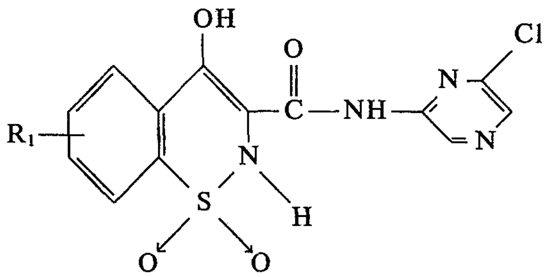

(IV)

in der $R_1$ die oben angegebenen Bedeutungen aufweist, mit einem Alkylhalogenid der allgemeinen Formel V,

$R_{11}$—Hal (V)

in der Hal ein Halogenatom und $R_{11}$ eine Methyl-, Äthyl- oder n-Propylgruppe bedeutet, in Gegenwart einer Base bei Temperaturen zwischen 0 und 80°C behandelt wird, und gegebenenfalls vor der Durchführung dieser Verfahren die 4-Hydroxygruppe in Verbindungen der allgemeinen Formeln II oder IV geschützt wird, wobei nach der Beendigung der Umsetzung die Schutzgruppe wieder abgespalten wird, und gewünschtenfalls eine nach den obigen Verfahren erhaltene Verbindung der allgemeinen Formel I mittels einer anorganischen oder organischen Base anschließend in ihr Salz übergeführt wird.

5. Verfahren nach Anspruch 4a, dadurch gekennzeichnet, daß ein 4-Hydroxy-2H-1,2-benzothiazin-1,1-dioxid-3-carbonsäurederivat der allgemeinen Formel II, in der X eine Alkoxygruppe mit 1 bis 8 Kohlenstoffatomen, eine Phenylalkoxygruppe mit insgesamt 7 bis 10 Kohlenstoffatomen, die Phenyloxygruppe, ein Halogenatom, die freie Aminogruppe, eine Alkylaminogruppe mit 1 bis 8 Kohlenstoffatomen, eine Cycloalkylaminogruppe mit 3 bis 10 Kohlenstoffatomen, eine Phenylalkylaminogruppe mit insgesamt 7 bis 10 Kohlenstoffatomen oder die Anilinogruppe bedeutet und $R_1$ und $R_2$ wie in Anspruch 4 definiert sind, mit dem 2-Amino-6-chlorpyrazin umgesetzt wird und, sofern X in der allgemeinen Formel II eine Alkoxygruppe bedeutet, der entstehende entsprechende Alkohol durch azeotrope Destillation entfernt wird.

6. Verfahren nach Anspruch 4b, dadurch gekennzeichnet, daß als Basen Alkali- oder Erdalkalihydroxide, -carbonate oder -alkoholate oder tertiäre Amine in wäßrigem, alkoholischem oder wäßrig-alkoholischem Medium oder Alkali- oder Erdalkalimetallhydride in aprotischen organischen Lösungsmitteln verwendet werden, wobei bei Verwendung von Alkali- oder Erdalkalicarbonaten auch aliphatische Ketone als Lösungsmittel in Frage kommen.

7. Verfahren nach Anspruch 4a, dadurch gekennzeichnet, daß man, sofern X in der allgemeinen Formel II die Aminogruppe oder eine Alkylamino-, Cycloalkylamino-, Phenylalkylamino- oder Anilinogruppe bedeutet, das Pyridin der Formel III im Überschuß einsetzt und bei der Umsetzung katalytische Mengen von p-Toluolsulfonsäure zusetzt.

8. Verfahren nach Anspruch 4a und 4b, dadurch gekennzeichnet, daß die freie 4-Hydroxygruppe in einer Verbindung der allgemeinen Formeln II und IV vor der Umsetzung durch Verätherung in eine Alkoxy- oder Phenylalkoxygruppe übergeführt wird und nach der Umsetzung eine derartige Schutzgruppe durch Erhitzen in Mineralsäuren auf Temperaturen bis zu 100°C oder durch Zugabe von Bortrihalogeniden bei Temperaturen zwischen −80°C und +80°C wieder abgespalten wird.

9. Arzneimittel, enthaltend eine oder mehrere Verbindungen der allgemeinen Formel I gemäß Anspruch 1 und übliche Träger- und/oder Zusatzstoffe.

10. Verwendung einer Verbindung der allgemeinen Formel I gemäß Anspruch 1 oder ihrer physiologisch verträglichen Salze zur Herstellung von Arzneimitteln mit antithrombotischer Wirkung.

**Patentansprüche für den Vertragsstaat: AT**

1. Verfahren zur Herstellung von neuen 4-Hydroxy-2H-1,2-benzothiazin-3-carboxamid-1,1-dioxiden der allgemeinen Formel I

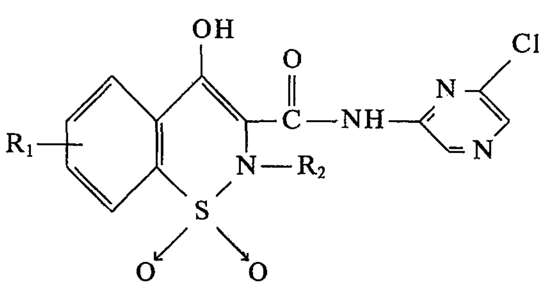

(I)

in der

$R_1$ ein Wasserstoffatom, die Methyl- oder Methoxygruppe oder ein Fluor- oder Chloratom und
$R_2$ ein Wasserstoffatom oder eine Methyl-, Äthyl- oder n-Propylgruppe bedeuten,

und von deren Salzen mit anorganischen oder organischen Basen, dadurch gekennzeichnet, daß

a) ein 4-Hydroxy-2H-1,2-benzothiazin-1,1-dioxid-3-carbonsäurederivat der allgemeinen Formel II,

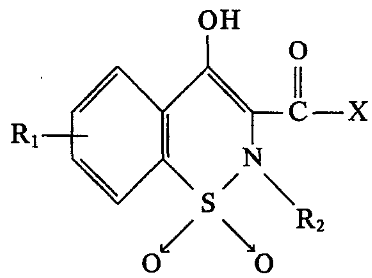

(II)

in der X eine nucleophil austauschbare Gruppe bedeutet und $R_1$ und $R_2$ wie oben definiert sind, mit 2-Amino-6-chlor-pyrazin der Formel III

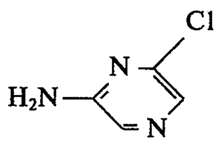

(III)

in einem indifferenten organischen Lösungsmittel oder in einem Überschuß des Pyrazins der Formel III bei Temperaturen zwischen 20 und 200°C umgesetzt wird oder

b) zur Herstellung von Verbindungen der allgemeinen Formel I, in der $R_2$ die Methyl-, Äthyl- oder n-Propylgruppe bedeutet und $R_1$ wie eingangs definiert ist, ein 4-Hydroxy-2H-1,2-benzothiazin-3-carboxamid der allgemeinen Formel IV,

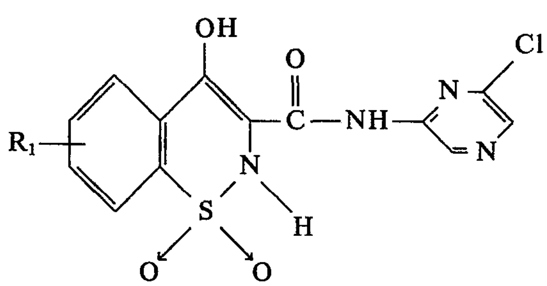

(IV)

in der $R_1$ die oben angegebenen Bedeutungen aufweist, mit einem Alkylhalogenid der allgemeinen Formel V,

$R_{11}$—Hal (V)

in der Hal ein Halogenatom und $R_{11}$ eine Methyl-, Äthyl- oder n-Propylgruppe bedeutet, in Gegenwart einer Base bei Temperaturen zwischen 0 und 80°C behandelt wird, und gegebenenfalls vor der Durchführung dieser Verfahren die 4-Hydroxygruppe in Verbindungen der allgemeinen Formeln II oder IV geschützt wird, wobei nach der Beendigung der Umsetzung die Schutzgruppe wieder abgespalten wird, und gewünschtenfalls eine nach den obigen Verfahren erhaltene Verbindung der allgemeinen Formel I mittels einer anorganischen oder organischen Base anschließend in ihr Salz übergeführt wird.

2. Verfahren nach Anspruch 1a, dadurch gekennzeichnet, daß, ein 4-Hydroxy-2H-1,2-benzothiazin-1,1-dioxid-3-carbonsäurederivat der allgemeinen Formel II, in der X eine Alkoxygruppe mit 1 bis 8 Kohlenstoffatomen, eine Phenylalkoxygruppe mit insgesamt 7 bis 10 Kohlenstoffatomen, die Phenyloxygruppe, ein Halogenatom, die freie Aminogruppe, eine Alkylaminogruppe mit 1 bis 8 Kohlenstoffatomen, eine Cycloalkylaminogruppe mit 3 bis 10 Kohlenstoffatomen, eine Phenylalkylaminogruppe mit insgesamt 7 bis 10 Kohlenstoffatomen oder die Anilinogruppe bedeutet und $R_1$ und $R_2$ wie in Anspruch 1 definiert sind, mit dem 2-Amino-6-chlorpyrazin umgesetzt wird und, sofern X in der allge-

meinen Formel II eine Alkoxygruppe bedeutet, der entstehende entsprechende Alkohol durch azeotrope Destillation entfernt wird.

3. Verfahren nach Anspruch 1b, dadurch gekennzeichnet, daß als Basen Alkali- oder Erdalkalihydroxide, -carbonate oder -alkoholate oder tertiäre Amine in wäßrigem, alkoholischem oder wäßrig-alkoholischem Medium oder Alkali- oder Erdalkalimetallhydride in aprotischen organischen Lösungsmitteln verwendet werden, wobei bei Verwendung von Alkali- oder Erdalkalicarbonaten auch aliphatische Ketone als Lösungsmittel in Frage kommen.

4. Verfahren nach Anspruch 1a, dadurch gekennzeichnet, daß man, sofern X in der allgemeinen Formel II die Aminogruppe oder eine Alkylamino-, Cycloalkylamino-, Phenylalkylamino- oder Anilinogruppe bedeutet, das Pyridin der Formel III im Überschuß einsetzt und bei der Umsetzung katalytische Mengen von p-Toluolsulfonsäure zusetzt.

5. Verfahren nach Anspruch 1a und 1b, dadurch gekennzeichnet, daß die freie 4-Hydroxygruppe in einer Verbindung der allgemeinen Formeln II und IV vor der Umsetzung durch Verätherung in eine Alkoxy- oder Phenylalkoxygruppe übergeführt wird und nach der Umsetzung eine derartige Schutzgruppe durch Erhitzen in Mineralsäuren auf Temperaturen bis zu 100°C oder durch Zugabe von Bortrihalogeniden bei Temperaturen zwischen −80°C und +80°C wieder abgespalten wird.

**Claims for the contracting states: BE, CH, DE, FR, IT, LI, LU, NL, SE**

1. 4-Hydroxy-2H-1,2-benzothiazine-3-carboxamide-1,1-dioxides of general formula I

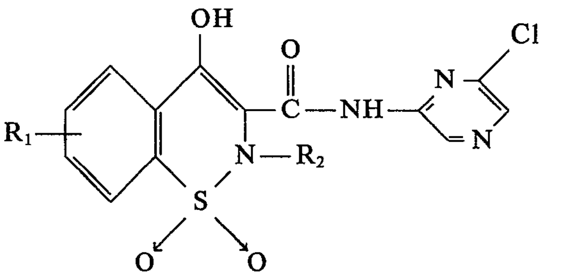

(I)

wherein

$R_1$ represents a hydrogen atom, a methyl or methoxy group or a fluorine or chlorine atom and
$R_2$ represents a hydrogen atom or a methyl, ethyl or n-propyl group,

and the physiologically acceptable salts thereof with inorganic or organic bases.

2. N-(6-Chloro-pyrazin-2-yl)-4-hydroxy-2-methyl-2H-1,2-benzothiazine-3-carboxamide-1,1dioxide and the physiologically acceptable salts thereof with inorganic or organic bases.

3. The sodium salts of the 4-hydroxy-2H-1,2-benzothiazine-3-carboxamide-1,1-dioxides of general formula I as claimed in claim 1.

4. Process for preparing 4-hydroxy-2H-1,2-benzothiazine-3-carboxamide-1,1-dioxides as claimed in claim 1 and the salts thereof with inorganic or organic bases, characterised in that

a) a 4-hydroxy-2H-1,2-benzothiazine-1,1-dioxide-3-carboxylic acid derivative of general formulaII

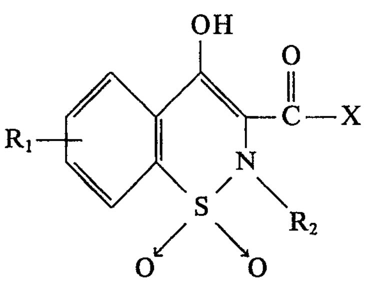

(II)

wherein X represents a nucleophilically exchangeable group and $R_1$ and $R_2$ are as hereinbefore defined, is reacted with 2-amino-6-chloro-pyrazine of formula III

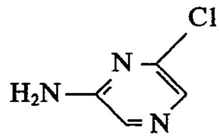

(III)

in an inert organic solvent or in an excess of the pyrazine of formula III at temperatures of between 20 and 200°C, or

b) in order to prepare compounds of general formula I wherein $R_2$ represents a methyl, ethyl or n-propyl group and $R_1$ is as hereinbefore defined, a 4-hydroxy-2H-1,2-benzothiazine-3-carboxamide of general formula IV

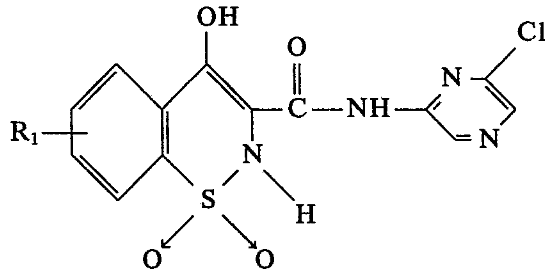

(IV)

wherein $R_1$ is as hereinbefore defined, is treated with an alkyl halide of general formula V

$R_{11}$—Hal (V)

wherein Hal represents a halogen atom and $R_{11}$ represents a methyl, ethyl or N-propyl group, in the presence of a base at temperatures of between 0 and 80°C, and optionally before these processes are carried out the 4-hydroxy group in compounds of general formula II or IV is protected and after the reaction has ended the protecting group is split off again, and if desired a compound of general formula I obtained by the above methods is subsequently converted into a salt thereof by means of an inorganic or organic base.

5. Process as claimed in claim 4a, characterised in that a 4-hydroxy-2H-1,2-benzothiazine-1,1-dioxide-3-carboxylic acid derivative of general formula II wherein X represents an alkoxy group with 1 to 8 carbon atoms, a phenylalkoxy group with a total of 7 to 10 carbon atoms, a phenyloxy group, a halogen atom, a free amino group, an alkylamino group with 1 to 8 carbon atoms, a cycloalkylamino group with 3 to 10 carbon atoms, a phenylalkylamino group with a total of 7 to 10 carbon atoms or an anilino group and $R_1$ and $R_2$ are defined as in claim 4 is reacted with 2-amino-6-chloropyrazine and, if X in general formula II represents an alkoxy group, the corresponding alcohol obtained is removed by azeotropic distillation.

6. Process as claimed in claim 4b, characterised in that the bases used are alkali or alkaline earth metal hydroxides, carbonates or alkoxides or tertiary amines in an aqueous, alcoholic or aqueous-alcoholic medium or alkali or alkaline earth metal hydrides in aprotic organic solvents, whilst if alkali or alkaline earth metal carbonates are used aliphatic ketones may also be used as the solvent.

7. Process as claimed in claim 4, characterised in that, if X in general formula II represents an amino group or an alkylamino, cycloalkylamino, phenylalkylamino or anilino group, the pyridine of formula III is used in excess and catalytic quantities of p-toluenesulphonic acid are added during the reaction.

8. Process as claimed in claim 4a and 4b, characterised in that the free 4-hydroxy group in a compound of general formula II or IV is converted by etherification into an alkoxy or phenylalkoxy group before the reaction and, after the reaction, a protecting group of this kind is split off again by heating in mineral acids to temperatures of up to 100°C or adding boron trihalides at temperatures of between −80°C and +80°C.

9. Pharmaceutical compositions containing one or more compounds of general formula I as claimed in claim 1 and conventional carriers and/or additives.

10. Use of a compound of general formula I as claimed in claim 1 or a physiologically acceptable salt thereof for the preparation of pharmaceutical compositions with an antithrombotic activity.

**Claims for the contracting state: AT**

1. Process for preparing new 4-hydroxy-2H-1,2-benzothiazine-3-carboxamide-1,1-dioxides of general formula

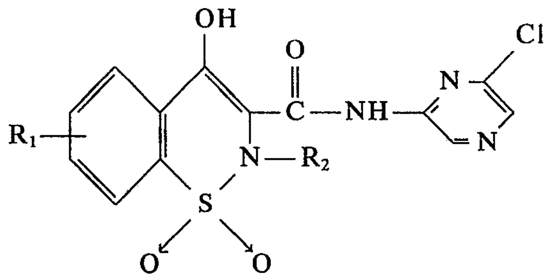

(I)

wherein

$R_1$ represents a hydrogen atom, a methyl or methoxy group or a fluorine or chlorine atom and
$R_2$ represents a hydrogen atom or a methyl, ethyl or n-propyl group,

and the salts thereof with inorganic or organic bases, characterised in that

a) a 4-hydroxy-2H-1,2-benzothiazine-1,1-dioxide-3-carboxylic acid derivative of general formula II

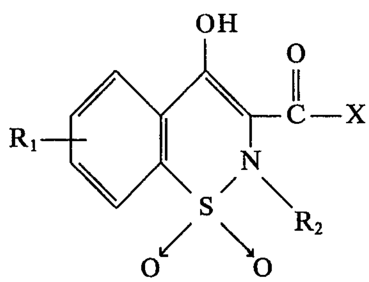

(II)

wherein X represents a nucleophilically exchangeable group and $R_1$ and $R_2$ are as hereinbefore defined, is reacted with 2-amino-6-chloro-pyrazine of formula III

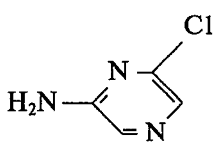

(III)

in an inert organic solvent or in an excess of the pyrazine of formula III at temperatures of between 20 and 200°C, or

b) in order to prepare compounds of general formula I wherein $R_2$ represents a methyl, ethyl or n-propyl group and $R_1$ is as hereinbefore defined, a 4-hydroxy-2H-1,2-benzothiazine-3-carboxamide of general formula IV

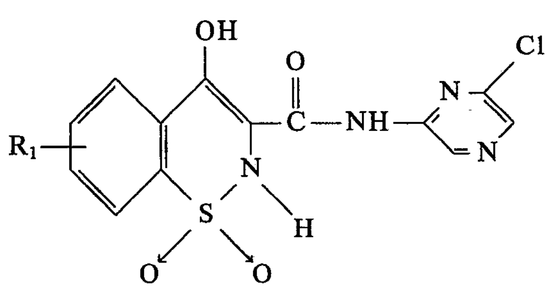

(IV)

wherein $R_1$ is as hereinbefore defined, is treated with an alkyl halide of general formula V

$R_{11}$—Hal (V)

wherein Hal represents a halogen atom and $R_{11}$ represents a methyl, ethyl or N-propyl group, in the presence of a base at temperatures of between 0 and 80°C, and optionally before these processes are carried out the 4-hydroxy group in compounds of general formula II or IV is protected and after the reaction has ended the protecting group is split off again, and if desired a compound of general formula I obtained by the above methods is subsequently converted into a salt thereof by means of an inorganic or organic base.

2. Process as claimed in claim 1a, characterised in that a 4-hydroxy-2H-1,2-benzothiazine-1,1-dioxide-3-carboxylic acid derivative of general formula II wherein X represents an alkoxy group with 1 to 8 carbon atoms, a phenylalkoxy group with a total of 7 to 10 carbon atoms, a phenyloxy group, a halogen atom, a free amino group, an alkylamino group with 1 to 8 carbon atoms, a cycloalkylamino group with 3 to 10 carbon atoms, a phenylalkylamino group with a total of 7 to 10 carbon atoms or an anilino group and $R_1$ and $R_2$ are defined as in claim 1 is reacted with 2-amino-6-chloropyrazine and, if X in general formula II represents an alkoxy group, the corresponding alcohol obtained is removed by azeotropic distillation.

3. Process as claimed in claim 1b, characterised in that the bases used are alkali or alkaline earth metal hydroxides, carbonates or alkoxides or tertiary amines in an aqueous, alcoholic or aqueous-alcoholic medium or alkali or alkaline earth metal hydrides in aprotic organic solvents, whilst if alkali or alkaline earth metal carbonates are used aliphatic ketones may also be used as the solvent.

4. Process as claimed in claim 1, characterised in that, if X in general formula II represents an amino group or an alkylamino, cycloalkylamino, phenylalkylamino or anilino group, the pyridine of formula III is used in excess and catalytic quantities of p-toluenesulphonic acid are added during the reaction.

5. Process as claimed in claim 1a and 1b, characterised in that the free 4-hydroxy group in a compound of general formula II or IV is converted by etherification into an alkoxy or phenylalkoxy group before the reaction and, after the reaction, a protecting group of this kind is split off again by heating in mineral acids to temperatures of up to 100°C or adding boron trihalides at temperatures of between −80°C and +80°C.

**Revendications pour les états contractants: BE, CH, DE, FR, IT, LI, LU, NL, SE**

1. 1,1-dioxydes de 4-hydroxy-2H-1,2-benzothiazine-3-carboxamide de formule générale I,

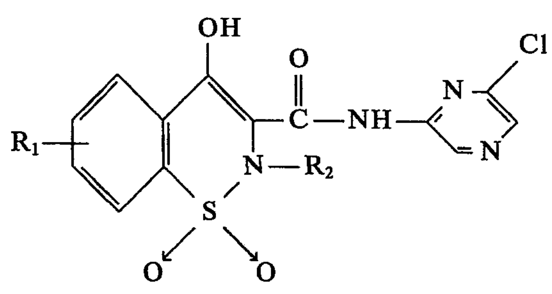

(I)

dans laquelle

$R_1$ représente un atome d'hydrogène, le groupe méthyle ou méthoxy ou un atome de fluor ou de clore, et
$R_2$ représente un atome d'hydrogène ou un groupe méthyle, éthyle ou n-propyle,

et leurs sels physiologiquement supportables avec des bases minérales ou organiques.

2. Le 1,1-dioxyde de N-(6-chloro-pyrazine-2-yl)-4-hydroxy-2-méthyl-2H-1,2-benzothiazine-3-carboxamide et ses sels physiologiquement supportables avec des bases minérales ou organiques.

3. Les sels de sodium des 1,1-dioxydes de 4-hydroxy-2H-1,2-benzothiazine-3-carboxamide de formule générale I selon la revendication 1.

4. Procédé pour la préparation de 1,1-dioxydes de 4-hydroxy-2H-1,2-benzothiazine-3-carboxamide selon la revendication 1 et de leurs sels avec des bases minérales ou organiques, caractérisé en ce que

a) on fait réagir un dérivé d'acide 4-hydroxy-2H-1,2-benzothiazine-1,1-dioxyde-3-carboxylique de formule générale II,

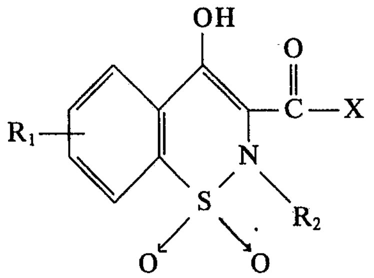

(II)

dans laquelle X est un groupe échangeable de façon nucléophile, et $R_1$ et $R_2$ sont définis comme plus haut, avec la 2-amino-6-chloro-pyrazine de formule III

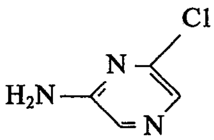

(III)

dans un solvant organique indifférent ou dans un excès de la pyrazine de formule III à des températures entre 20 et 200°C ou

b) pour la préparation de composés de formule générale I dans laquelle $R_2$ représente le groupe méthyle, éthyle ou n-propyle et $R_1$ est défini comme au début, on traite un 4-hydroxy-2H-1,2-benzothiazine-3-carboxamide de formule générale IV,

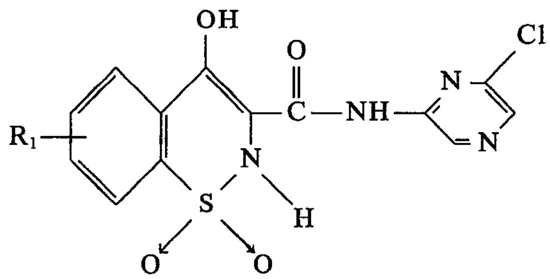

(IV)

dans laquelle $R_1$ présente les significations indiquées plus haut, au moyen d'un halogénure d'alcoyle de formule générale V,

$R_{11}$—Hal (V)

dans laquelle Hal représente un atome d'halogène et $R_{11}$ un groupe méthyle, éthyle ou n-propyle, en présence d'une base à des températures entre 0 et 80°C, et éventuellement avant la mise en œuvre de ce procédé, on protège le groupe 4-hydroxy dans les composés de formules générales II ou IV, lorsque la réaction est terminée, le groupe protecteur étant de nouveau clivé, et si on le désire, on transforme un composé de formule générale I obtenu selon le procédé ci-dessus au moyen d'une base minérale ou organique ensuite en son sel.

5. Procédé selon la revendication 4a, caractérisé en ce qu'on fait réagir un dérivé d'acide 4-hydroxy-2H-1,2-benzothiazine-1,1-dioxyde-3-carboxylique de formule générale II, dans laquelle X représente un groupe alcoxy avec 1 à 8 atomes de carbone, un groupe phénylalcoxy avec en tout 7 à 10 atomes de carbone, le groupe phényloxy, un atome d'halogène, le groupe amino libre, un groupe alcoylamino avec 1 à 8 atomes de carbone, un groupe cycloalcoylamino avec 3 à 10 atomes de carbone, un groupe phénylalcoylamino avec en tout 7 à 10 atomes de carbone ou le groupe anilino et $R_1$ et $R_2$ sont définis comme dans la revendication 4, avec la 2-amino-6-chloro-pyrazine et, dans la mesure où X dans la formule générale II représente un groupe alcoxy, on élimine l'alcool résultant correspondant par distillation azéotropique.

6. Procédé selon la revendication 4b, caractérisé en ce qu'on utilise comme bases des hydroxydes, carbonates ou alcoolates alcalins ou alcalino-terreux ou des amines tertiaires en milieu aqueux, alcoolique ou hydroalcoolique ou des hydrures de métaux alcalins ou alcalino-terreux dans des solvants organiques aprotiques, des cétones aliphatiques entrant également en ligne de compte en tant que solvants, lorsque l'on utilise des carbonates alcalins ou alcalino-terreux.

7. Procédé selon la revendication 4a, caractérisé en ce que, dans la mesure où X dans la formule générale II représente le groupe amino ou un groupe alcoylamino, cycloalcoylamino, phénylalcoylamino ou anilino, on met en œuvre la pyridine de formule III en excès et on ajoute, lors de la réaction, des quantités catalytiques d'acide p-toluène sulfonique.

8. Procédé selon la revendication 4a et 4b, caractérisé en ce que le groupe 4-hydroxy libre dans un composé de formules générales II et IV est transformé avant la réaction, par éthérification, en un groupe alcoxy ou phénylalcoxy et en ce qu'après la réaction, un tel groupe protecteur est de nouveau clivé par chauffage dans des acides minéraux à des températures allant jusqu'à 100°C ou par addition de trihalogénures de bore, à des températures entre −80°C et +80°C.

9. Médicament contenant un ou plusieurs composés de formule générale I selon la revendication 1 et des excipients et/ou additifs usuels.

10. Utilisation d'un composé de formule générale I selon la revendication 1 ou de ses sels physiologiquement supportables pour la préparation de médicaments avec une activité antithrombotique.

**Revendications pour l'état contractant: AT**

1. Procédé pour la préparation de nouveaux 1,1-dioxydes de 4-hydroxy-2H-1,2-benzothiazine-3-carboxamide de formule générale I

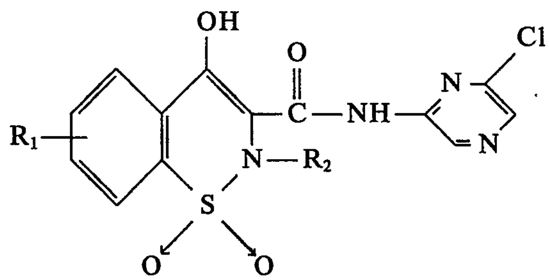

(I)

dans laquelle

$R_1$ représente un atome d'hydrogène, le groupe méthyle ou méthoxy ou un atome de fluor ou de chlore, et

$R_2$ représente un atome d'hydrogène ou un groupe méthyle, éthyle ou n-propyle,

et de leurs sels avec des bases minérales ou organiques, caractérisé en ce que

a) on fait réagir un dérivé d'acide 4-hydroxy-2H-1,2-benzothiazine-1,1-dioxyde-3-carboxylique de formule générale II,

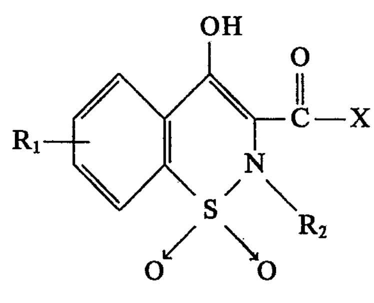

(II)

dans laquelle X représente un groupe échangeable de façon nucléophile, et $R_1$ et $R_2$ sont définis comme plus haut, avec la 2-amino-6-chloro-pyrazine de formule III

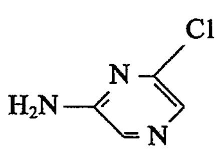

(III)

dans un solvant organique indifférent ou dans un excès de la pyrazine de formule III à des températures entre 20 et 200°C ou

b) pour la préparation de composés de formule générale I dans laquelle $R_2$ représente le groupe méthyle, éthyle ou n-propyle et $R_1$ est défini comme au début, on traite un 4-hydroxy-2H-1,2-benzothiazine-3-carboxamide de formule générale IV,

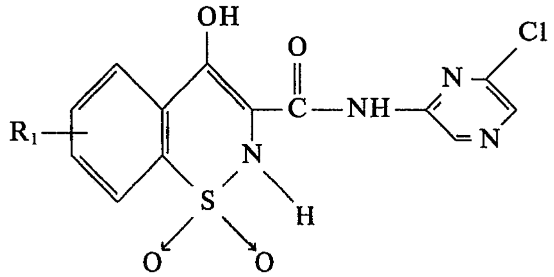

(IV)

dans laquelle $R_1$ présente les significations indiquées plus haut, au moyen d'un halogénure d'alcoyle de formule générale V,

$R_{11}$ —Hal (V)

dans laquelle Hal représente un atome d'halogène et $R_{11}$ représente un groupe méthyle, éthyle ou n-propyle, en présence d'une base à des températures entre 0 et 80°C, et éventuellement avant la mise en œuvre de ce procédé, on protège le groupe 4-hydroxy dans les composés de formules générales II ou IV, lorsque la réaction est terminée, le groupe protecteur étant de nouveau clivé, et si on le désire, on transforme un composé de formule générale I obtenu selon les procédés ci-dessus au moyen d'une base minérale ou organique, ensuite en son sel.

2. Procédé selon la revendication 1a, caractérisé en ce qu'on fait réagir un dérivé d'acide 4-hydroxy-2H-1,2-benzothiazine-1,1-dioxyde-3-carboxylique de formule générale II, dans laquelle X représente un groupe alcoxy avec 1 à 8 atomes de carbone, un groupe phénylalcoxy avec en tout 7 à 10 atomes de carbone, le groupe phényloxy, un atome d'halogène, le groupe amino libre, un groupe alcoylamino avec 1 à 8 atomes de carbone, un groupe cycloalcoylamino avec 3 à 10 atomes de carbone, un groupe phénylalcoylamino avec en tout 7 à 10 atomes de carbone ou le groupe anilino et $R_1$ et $R_2$ sont définis comme dans la revendication 1, avec la 2-amino-6-chloro-pyrazine et, dans la mesure où X dans la formule générale II représente un groupe alcoxy, on élimine l'alcool résultant correspondant par distillation azéotropique.

3. Procédé selon la revendication 1b, caractérisé en ce qu'on utilise comme bases des hydroxydes, carbonates ou alcoolates alcalins ou alcalino-terreux ou des amines tertiaires en milieu aqueux, alcoolique ou hydroalcoolique ou des hydrures de métaux alcalins ou alcalino-terreux dans des solvants organiques aprotiques, des cétones aliphatiques entrant également en ligne de compte en tant que solvants, lorsque l'on utilise des carbonates alcalins ou alcalino-terreux.

4. Procédé selon la revendication 1a, caractérisé en ce que, dans la mesure où X dans la formule générale II représente le groupe amino ou un groupe alcoylamino, cycloalcoylamino, phénylalcoylamino ou anilino, on met en œuvre la pyridine de formule III en excès et on ajoute, lors de la réaction, des quantités catalytiques d'acide p-toluènesulfonique.

5. Procédé selon la revendication 1a et 1b, caractérisé en ce que le groupe 4-hydroxy libre dans un composé de formules générales II et IV est transformé avant la réaction, par éthérification, en un groupe alcoxy ou phénylalcoxy et en ce qu'après la réaction, un tel groupe protecteur est de nouveau clivé par chauffage dans des acides minéraux à des températures allant jusqu'à 100°C ou par addition de trihalogénures de bore, à des températures entre −80°C et +80°C.